# EUROPEAN PATENT SPECIFICATION

(11) **EP 1 621 889 B1**
(45) Date of publication and mention of the grant of the patent: **17.09.2008**
(21) Application number: 03717708.6
(22) Date of filing: 23.04.2003
(51) Int. Cl.: G01N 33/53, G01N 33/58

(54) **METHOD FOR DETECTING BINDING OF NUCLEIC ACID AND NUCLEIC ACID BINDING PROTEIN BY MONOMOLECULAR FLUORESCENT ANALYSYIS**
VERFAHREN ZUM NACHWEIS DER BINDUNG VON NUKLEINSÄURE UND NUKLEINSÄURE BINDENDEM PROTEIN MITTELS MONOMOLEKULARER FLUORESZENZANALYSE
PROCEDE DE DETECTION DE LIAISON ENTRE UN ACIDE NUCLEIQUE ET UNE PROTEINE DE LIAISON D'ACIDES NUCLEIQUES PAR L'ANALYSE DE FLUORESCENCE A MOLECULE ISOLEE

(43) Date of publication of application: 01.02.2006
(73) Proprietor: Olympus Corporation, Tokyo 151-0072 (JP)
(72) Inventor: KATO, Noriko, Hachioji-shi, Tokyo 192-0046 (JP); OKAMOTO, Naoaki, Hachioji-shi, Tokyo (JP)
(74) Representative: von Hellfeld, Axel
(86) International application number: PCT/JP2003/005189
(87) International publication number: WO 2004/095029

(56) References cited:
- JP-A- 11 004 638
- JP-A- 2001 272 404
- JP-A- 2002 281 965
- JP-A- 2003 035 714
- SEVENICH F W ET AL: "DNA binding and oligomerization of NtrC studied by fluorescence anisotropy and fluorescence correlation spectroscopy." 15 March 1998 (1998-03-15), NUCLEIC ACIDS RESEARCH. 15 MAR 1998, VOL. 26, NR. 6, PAGE(S) 1373 - 1381 , XP002384023 ISSN: 0305-1048 * figure 3 * * page 1373, column 2, line 15 - page 1374, column 1, line 27 *
- AUER M ET AL: "FLUORESCENCE CORRELATION SPECTROSCOPY: LEAD DISCOVERY BY MINIATURIZED HTS" DDT - DRUG DISCOVERY TODAY, ELSEVIER SCIENCE LTD, GB, vol. 3, no. 10, 1 October 1998 (1998-10-01), pages 457-465, XP000973192 ISSN: 1359-6446
- STERRER S ET AL: "FLUORESCENCE CORRELATION SPECTROSCOPY (FCS) - A HIGHLY SENSITIVE METHOD TO ANALYZE DRUG/TARGET INTERACTIONS" JOURNAL OF RECEPTOR AND SIGNAL TRANSDUCTION RESEARCH, MARCEL DEKKER, NEW YORK, NY, US, vol. 17, no. 1/3, 1997, pages 511-520, XP000671058 ISSN: 1079-9893
- KASK P ET AL: "FLUORESCENCE-INTENSITY DISTRIBUTION ANALYSIS AND ITS APPLICATION INBIOMOLECULAR DETECTION TECHNOLOGY" 23 November 1999 (1999-11-23), PROCEEDINGS OF THE NATIONAL ACADEMY OF SCIENCES OF USA, NATIONAL ACADEMY OF SCIENCE, WASHINGTON, DC, US, PAGE(S) 13756-13761 , XP001005935 ISSN: 0027-8424
- SCHWILLE P ET AL: "DUAL-COLOR FLUORESCENT CROSS-CORRELATION SPECTROSCOPY FOR MULTICOMPONENT DIFFUSIONAL ANALYSIS IN SOLUTION" April 1997 (1997-04), BIOPHYSICAL JOURNAL, NEW YORK, US, US, PAGE(S) 1878-1886 , XP008008967 ISSN: 0006-3495
- PALO K ET AL: "FLUORESCENCE INTENSITY MULTIPLE DISTRIBUTIONS ANALYSIS: CONCURRENT DETERMINATION OF DIFFUSION TIMES AND MOLECULAR BRIGHTNESS" BIOPHYSICAL JOURNAL, NEW YORK, US, US, vol. 79, no. 6, December 2000 (2000-12), pages 2858-2866, XP001005981 ISSN: 0006-3495
- MAGDE D ET AL: "Fluorescence correlation spectroscopy. II. An experimental realization." January 1974 (1974-01), BIOPOLYMERS. JAN 1974, VOL. 13, NR. 1, PAGE(S) 29 - 61 , XP002384024 ISSN: 0006-3525

## Description

### Technical field

The present invention relates to a method for detecting binding of a nucleic acid and a nucleic acid binding protein by monomolecular fluorescent analysis. In particular, the present invention is useful in analyzing protein function and detecting a drug utilizing it, by searching interaction between a gene and a transcription factor binding thereto.

### Background Art

Life phenomenon is carried out by normal expression and functioning of proteins synthesized by gene expression, and abnormal gene expression becomes a cause for carcinogenesis and apoptosis. A majority of gene expression is controlled by a DNA binding protein called transcription factor. A total picture of transcription system is being revealed by development of transcription study, and similarity of fundamental transcription mechanism is shown in a eukaryote including yeast to a human. For example, a steric structure of a TBP (TATA box-binding protein)/TFIIA/TFIIB complex integrated on a promoter has been already elucidated, and new information is being accumulated regarding protein-protein interaction between basal transcription factors on a DNA strand and formation of a higher structure of DNA with a basal transcription factor. For this reason, analysis of molecular mechanism of transcription activation or inactivation by a transcription regulatory factor has been performed with a focus on a transcription system which is a base for transcription, and finally, it is desired to understand life phenomenon such as development, differentiation, proliferation and canceration through a network and a cascade of transcription control. Under such the circumstances, development of a detection system for detecting rapidly and specifically a reaction of binding of a transcription factor and DNA is expected.

Hitherto, as a system for detecting specific binding of a transcription factor and a particular DNA sequence, analysis using electrophoresis and an isotope (radioactive substance) such as a gel shift assay, an *in vitro* transcription assay and a footprinting method has been known.

The gel shift assay utilizes reduction in mobility of electrophoresis of a DNA binding protein by specific binding of a transcription factor and a particular DNA sequence. In this method, since a large amount of a few mg of a protein is necessary, electrophoresis needs a time and, further, a molecular weight of a protein complex bound to DNA requires analysis by autoradiography, much time and cost are required. In addition, even when a specific sample is found, it is difficult to recover the sample, and it is hardly extended to proteome analysis. In addition, since many proteins need to be screened, much time, cost and labor are required.

The *in vitro* transcription assay measures transcription factor activity based on specific binding of a transcription factor to the DNA sequence, by putting in a test tube a prescribed DNA sequence as a template, an RNA polymerase, a transcription factor, and a substrate NTP (base components G, A, C and U), and performing a RNA synthesis reaction therein. In this method, since it is necessary to detect a synthesized RNA with analysis by electrophoresis and subsequent autoradiography, much time, cost and labor are required.

The footprinting method determines a nucleotide sequence recognizing site on a DNA chain of a protein which binds to a specific site of DNA. To describe in detail, in this method, first, a 5' or 3'-end of a DNA fragment containing a binding site of a protein is labeled with ³²P, the labeled DNA fragment is treated with DNase to perform non-specific base cutting and, after gel electrophoresis, autoradiography is performed, thereby, bands of DNA fragments which have been separated due to a difference in a length by each one base are obtained. On the other hand, when DNase treatment is performed by binding a protein to the aforementioned DNA fragment, bases of a binding site is not cut, a band corresponding to it disappears in autoradiogram, and it looks in white on colored background. By comparing autoradiograms of both of them, a binding site of a protein and a nucleotide sequence corresponding to the binding site can be determined. In this method, since it is also necessary to perform analysis by electrophoresis and autoradiography, much time, cost and labor are required.

In addition, there is also a method of analyzing interaction of a protein and a ligand using the same principle as that of the footprinting method. This is utilized for detecting a change in conformation of a protein. By applying to a transcription complex, a change in conformation due to a moribund complex and a dead-end complex can be detected (See Nagai & Shimamoto (1997) Most conserved regions of the E. coli primary sigma factor are involved in interaction with RNA polymerase core enzyme. Genes Cells 2, 725-734). However, also in this method, since analysis by an isotope, electrophoresis and autoradiography is used, facility investment and specialists are required, and an experiment is very difficult.

The journal article "DNA Binding and Oligomerization of NtrC Studied by Fluorescence Anisotropy and Fluorescence Coloration Spectroscopy" by Sevenich et al., Nucleic Acids Research, Vol. 26, No. 6, pp 1373-1381, describes the use of fluorescence anisotropy and fluorescence correlation Spectroscopy measurements of rhodamine-labelled DNA oligonucleotide duplexes to determine equilibrium binding constants for DNA binding of the prokaryotic transcription activator protein NtrC.

### Disclosure of the Invention

In view of the aforementioned circumstances, an object of the present invention is to provide a novel method for detecting binding of a nucleic acid and a nucleic acid binding protein, which can solve all problems possessed by the detecting method using an isotope (radioactive substance) and electrophoresis such as a gel shift assay, an *in vitro* transcription assay and a footprinting method.

The present inventors found out a new method of simply and rapidly detecting a protein which can bind to a nucleic acid, using a non-radioactive substance as a detection marker, which resulted in completion of the present invention. The method of the present invention can solve problems possessed by the previous detection method such as a gel shift assay, an *in vitro* transcription assay and a footprinting method at once.

That is, the present invention provides the following means.
(1) A method for detecting binding of a nucleic acid and two kinds of nucleic acid binding proteins by monomolecular fluorescent analysis, comprising:
   a step of detecting binding of a nucleic acid and a first nucleic acid binding protein; and a step of, when binding of a nucleic acid and a first nucleic acid binding protein is detected in the above step, detecting binding of the resulting nucleic acid-protein complex and a second nucleic acid binding protein.
(2) A method for detecting binding of a nucleic acid and n kinds (n indicates an integer of 2 or more) of nucleic acid binding proteins by monomolecular fluorescent analysis, comprising:
   a step of detecting binding of a nucleic acid and a first nucleic acid binding protein; and
   a step of, when binding of a nucleic acid and (X-1) kinds of first to (X-1)<th> nucleic acid binding proteins is detected in the above step, repeating a step of detecting binding of the resulting nucleic acid-protein complex and a X<th> nucleic acid binding protein (n-1) times sequentially from X = 2 to X = n.
(3) The method according to one of items (1) or (2), further comprising a step of confirming binding of a nucleic acid and one or more kinds of nucleic acid binding proteins using an antibody against any one of nucleic acid binding proteins.
(4) The method according to any one of items (1) to (3), wherein the nucleic acid has a sequence of a protein-binding site.
(5) The method according to any one of items (1) to (4), wherein the nucleic acid binding protein is a TATA box binding protein.
(6) The method according to any one of items (1) to (5), wherein binding of the nucleic acid and the nucleic acid binding protein is detected with a light signal by binding the nucleic acid with a fluorescent substance, a luminescent substance, an enzyme luminescent substance, or a radioactive substance.
(7) The method according to any one of items (1) to (6), wherein the nucleic acid binding protein is a basal transcription factor, a transcription promoting factor, or a transcription inhibiting factor.

### Brief Description of Drawings

FIG. 1 is a view showing a relationship between a molecular weight of a protein and a translational diffusion time.
FIG. 2 is a view showing a relationship between a DNA size and a translational diffusion time.
FIG. 3 is a view showing one example of a fluorescence correlation analyzing apparatus used in the detection method of the present invention.

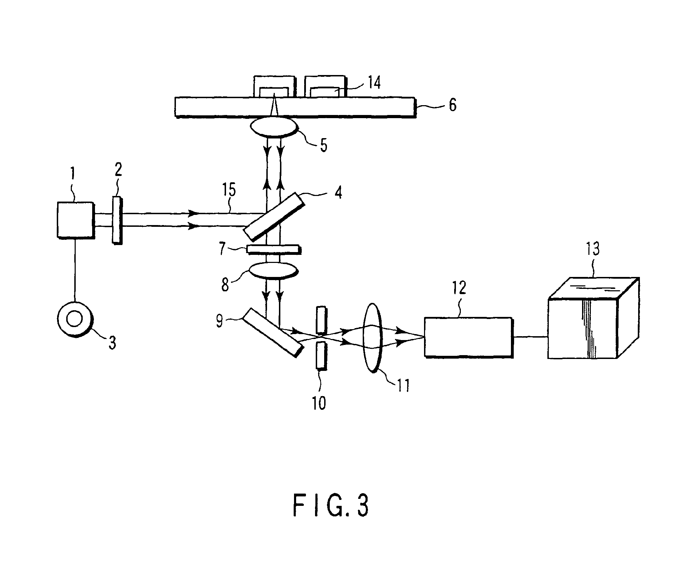

In FIG. 3, reference numerals 1 to 15 denote the following:
1 ... laser light source, 2... light intensity regulating means (ND filter), 3... light attenuation rate selecting apparatus (ND filter changer), 4... dichroic mirror, 5... objective lens, 6... stage, 7... filter, 8... tube lens, 9... reflection mirror, 10... pinhole, 11... lens, 12... light detector (avalanche photodiode), 13... fluorescent intensity recording means (computer), 14... sample, 15... light beam

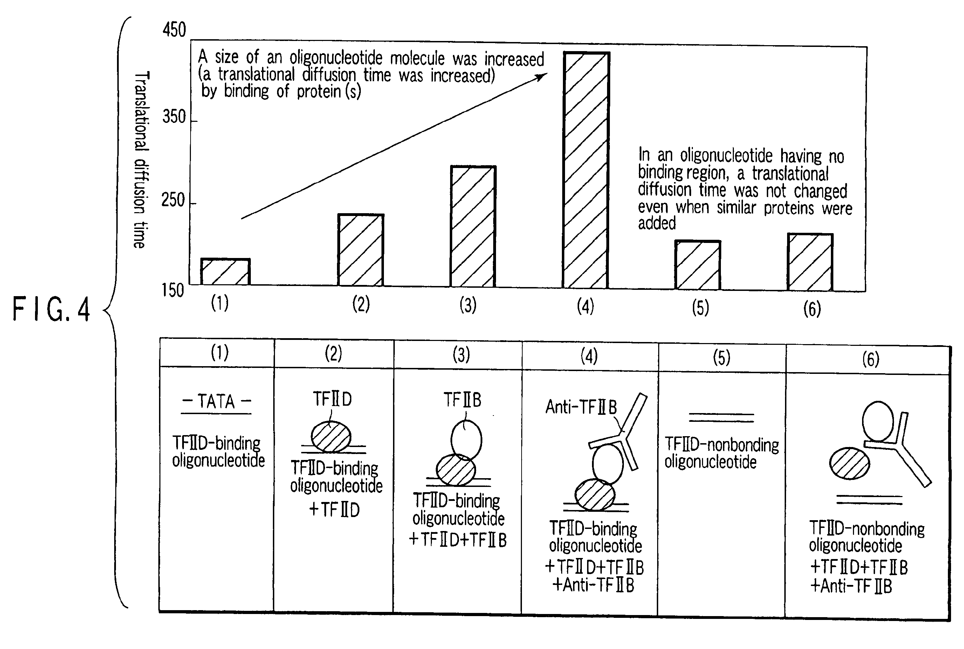

FIG. 4 is a view showing detection of interaction between DNA and a DNA binding protein.

### Best Mode for Carrying Out the Invention

### <Monomolecular fluorescent analysis technique>

First, monomolecular fluorescent analysis which is an analysis technique used in a method of the present invention will be explained.

Monomolecular fluorescent analysis is a technique of measuring a fluorescent signal (e.g. fluctuation movement and/or fluorescent intensity) derived from a fluorescent molecule which enters into and exists from a micro confocal region, and analyzing the obtained data by a function. This technique includes (1) analysis by Fluorescence Correlation Spectroscopy, (2) Fluorescence Intensity Distribution Analysis, and (3) Fluorescence Intensity Multiple Distribution Analysis performing these analyses simultaneously. Each of them will be explained below.
(1) Fluorescence Correlation Spectroscopy (hereinafter, also referred to as FCS) is a technique of measuring fluctuation movement of a fluorescence labeled target molecule in a medium, and correctly measuring a micromovement of individual target molecule by using Autocorrelation function (See D. Magde and E. Elson, "Fluorescence correlation spectroscopy. II. An experimental realization", Biopolymers 1974 13(1) 29-61.
   FCS is performed by analyzing a diffusion time from fluctuation of a fluorescent intensity by capturing Brownian movement of a fluorescent molecule in a micro-region in a solution with a laser confocal microscope, and determining a physical amount (the number and size of molecules). Analysis by FCS capturing molecular fluctuation in such the micro-region is effective for detecting intermolecular interaction at a high sensitivity and specifically.
   Principle of detection by FCS will be explained in more detail. In FCS, a fluorescent signal generated from a micro field region in a sample is detected and quantified with a microscope. Then, a fluorescence labeled target molecule in a medium is always moved (Brownian movement). Therefore, a detected fluorescent intensity is changed depending on a frequency of entrance of a target molecule into a micro field region, and a time during which the molecule stays in the region.
   For example, when an apparent molecular weight is increased by occurrence of dimerization of molecules, movement of a target molecule is slowed down, and the apparent molecular number is decreased. As a result, a frequency of entrance of a target molecule into a micro field region is reduced, and an observed fluorescent intensity is changed. By monitoring such the change in a fluorescent intensity, a change in an apparent molecular weight of a target molecule can be traced.
(2) Fluorescence Intensity Distribution Analysis (hereinafter, also referred to as FIDA) is disclosed in the references: P Kask, et al.; PNAS 23, 96, 13756-13761, 1999, and WO 98/16814. FIDA is a technique of irradiating a sample with laser, measuring the excitation light of a fluorescent molecule emitted from a sample with APD (high sensitive photodetector), and analyzing the measured fluorescent signal with double Poisson distribution function regarding photon count resolved per very short time of 40 microseconds, followed by statistical analysis. By FIDA, a fluorescent intensity per molecule (brightness; qn) and the number of a fluorescent molecule (cn) are calculated. Even when there is plurality of molecule types, they can be discriminated by distribution analysis, and a numerical value obtained by multiplying brightness with the molecule number regarding each molecule type can be calculated as a total fluorescent amount.
(3) Fluorescence Intensity Multiple Distribution Analysis (hereinafter, also referred to as FIMDA) performs FCS analysis and FIDA simultaneously.
Detailed contents are disclosed in the reference: K Palo, Biophysical Journal, 79, 2858-2866, 2000). According to FIMDA, data regarding a translational diffusion time of a fluorescent molecule, the number of molecules, and a fluorescent intensity per molecule (brightness) can be acquired simultaneously.

### <Detection method of the present invention>

Using the aforementioned monomolecular fluorescent analysis technique, detection of the present invention can be performed. That is, using the monomolecular fluorescent analysis technique, binding of a "free nucleic acid binding protein" to a nucleic acid to form a complex can be detected by increase in a translational diffusion time (i.e. increase in molecular weight) (see FIG. 4). In addition, using the monomolecular fluorescent analysis technique, a presence ratio of a "free nucleic acid binding protein" and a "nucleic acid-protein complex" obtained by binding with a nucleic acid can be obtained by a change in the number of molecules of each molecule, or a change in a fluorescent intensity per molecule.

Thereby, the detection method using the monomolecular fluorescent analysis technique of the present invention can detect not only the presence or the absence of binding of a nucleic acid binding protein to a nucleic acid, but also ability of a nucleic acid binding protein to bind to a nucleic acid (strength of binding force).

The method of the present invention will be explained in detail below.

The detection method of the present invention is a method of detecting binding of a nucleic acid and a nucleic acid binding protein by the monomolecular fluorescent analysis technique. In the detection method of the present invention, a nucleic acid binding protein may be one kind or a plurality of kinds.

That is, in the case of two kinds of nucleic acid binding proteins, the detection method using the monomolecular fluorescent analysis technique of the present invention includes:
a step of detecting binding of a nucleic acid and a first nucleic acid binding protein, and
a step of, when binding of a nucleic acid and a first nucleic acid binding protein is detected in the above step, detecting binding of the resulting nucleic acid-protein complex and a second nucleic acid binding protein.

Further, in the case of n kinds of nucleic acid binding proteins (n indicates integer of 2 or more), the detection method using monomolecular fluorescent analysis technique of the present invention includes:
a step of detecting binding of a nucleic acid and a first nucleic acid binding protein, and
a step of, when binding of a nucleic acid and (X-1) kinds of first to (X-1)^{th} nucleic acid binding proteins is detected in the above step, repeating a step of detecting binding of the resulting nucleic acid-protein complex and a X^{th} nucleic acid binding protein (n-1) times sequentially from X = 2 to X = n. 2 to 9 kinds are usually contemplated as n kinds.

According to the detection method of the present invention, by selecting a "nucleic acid" in advance, a "nucleic acid binding protein which binds to the nucleic acid" can be searched. In addition, according to the detection method of the present invention, by selecting a "nucleic acid binding protein" in advance, a "nucleic acid to which the nucleic acid binding protein binds" can be searched.

### (Regarding nucleic acid and nucleic acid binding protein)

In the present invention, a "nucleic acid" may be either DNA or RNA, and may contain a modified base. In addition, the nucleic acid may be single-stranded or double-stranded, but not particularly limited. When a "nucleic acid" is selected in advance and a "nucleic acid binding protein which binds to the nucleic acid" is searched, it is preferable that the "nucleic acid" is a nucleic acid having a protein-binding site. More specifically, the nucleic acid may be a nucleic acid containing a protein-binding site which initiates transcription of a gene or promotes or suppresses transcription by specific binding of a protein. As described, when a "nucleic acid" is selected in advance and a "nucleic acid binding protein which binds to the nucleic acid" is searched, the "nucleic acid binding protein" can be an arbitrary protein which is predicted to specifically or nonspecifically bind to the "nucleic acid".

In addition, a length of the "nucleic acid" is not particularly limited as far as a desired sequence (e.g. protein-binding site) is contained therein. However, when binding of a "nucleic acid" and a "nucleic acid binding protein" is detected based on a change in a molecular weight and the "nucleic acid binding protein" is labeled with fluorescence, it is desirable to set a molecular weight of the "nucleic acid" so that 5-fold or more increase in a molecular size is caused by binding of the "nucleic acid binding protein" and the "nucleic acid" to form a complex, as compared with a "free nucleic acid binding protein".

On the other hand, when a "nucleic acid binding protein" is selected in advance and a "nucleic acid to which the protein binds" is searched, the "nucleic acid binding protein" is not particularly limited as far as it is known to bind to a nucleic acid. Examples thereof include a transcription factor involved in transcription regulation such as initiation, elongation and termination of transcription by binding to DNA. More specifically, examples of a transcription factor include a basal transcription factor, a transcription promoting factor, and a transcription inhibiting factor. There are more than 50 kinds of the known transcription factors, and examples include AP-1 (activator protein 1), c-Myb, and FAST-1. It is said that a molecular weight of a transcription factor is 12 to 250 kDa, and there are many factors having a molecular weight of around 40 kDa. As a sequence to which a transcription factor binds, a short DNA is of 6 bases, and a nucleotide sequence which can be measured by monomolecular fluorescent analysis has preferably a length of up to 5200 base pairs, more preferably a length of up to 1000 base pairs. In this way, when a "nucleic acid binding protein" is selected in advance and a "nucleic acid to which the protein binds" is searched, the "nucleic acid" can be an arbitrary nucleic acid which is predicted to be specifically or nonspecifically be bound by the "nucleic acid binding protein".

When a plurality of "nucleic acid binding proteins" are predicted, each nucleic acid binding protein may bind to a nucleic acid directly, or may bind to a nucleic acid indirectly via other nucleic acid binding protein.

Examples of a "nucleic acid" selected in advance include a nucleic acid containing a TATA box. The TATA box is a particular DNA sequence which is situated upstream of a position of gene transcription initiation, and plays an important role in transcription initiation. A sequence of the TATA box is known as 5'-TATAA(or T)AT(or A)-3'. It is known that a TATA box-binding protein (i.e. transcription factor TFIID) binds to this TATA box and, thereafter, a transcription factor TFIIB binds thereto. According to the detection method of the present invention, a protein which can bind to a protein-binding site such as the TATA box can be searched.

In addition, when a TATA box-binding protein (i.e. TFIID) is used as a specific example of a "nucleic acid binding protein" selected in advance, a nucleic acid to which the TATA box-binding protein can bind can be searched according to the method of the present invention.

In the present invention, a "nucleic acid" and a "nucleic acid binding protein" may be those prepared by any method. Regarding the "nucleic acid", a desired sequence can be prepared by synthesis. Regarding the "nucleic binding protein", a nucleic acid binding protein may be obtained by *in vitro* expression by the known genetic engineering procedure using a gene encoding the protein. Alternatively, a fraction containing the protein may be extracted from *in vivo* or a cell. Alternatively, a sample obtained from a protein or a peptide library may be used.

The detection method of the present invention is not limited to the aforementioned specific description, and it goes without saying that it can be utilized for detection of binding of an arbitrary nucleic acid and an arbitrary nucleic acid binding protein.

Transcription of a eukaryote will be explained below.

In a eukaryote, three kinds of RNA polymerases are present, and each of them transcribes a different class of a gene. For transcribing a gene encoding a protein, RNA polymerase II and six kinds of basal transcription factors (TFIIA, TFIIB, TFIID, TFIIE, TFIIF, TFIIH) are necessary and, among these factors, only one factor having DNA binding ability is TFIID. TFIID binds to a TATA box, and subsequently respective factors are successively assembled on a promoter, thereby, a transcription initiation complex (PIC) is formed. Transcription proceeds to a transcription elongation stage by separation of a RNA polymerase from the PIC. In these a few years, respective factors constituting a transcription system have been screened, and mechanism of PIC formation has been elucidated in detail. Further, it has been also revealed that mutation in a basal transcription factor or a transcription elongation factor is closely associated with a human hereditary disease, and a useful detection method is desired. Therefore, the detection method of the present invention is also useful in investigating influence of mutation in a transcription factor on ability to bind to DNA.

### (Step of detecting binding of nucleic acid and nucleic acid binding protein)

In the present invention, it is necessary that at least one of the "nucleic acid binding protein" and the "nucleic acid" is labeled in advance for monomolecular fluorescent analysis. The cases (A) to (C) as a representative pattern of a labeling will be exemplified below, but the present invention is not limited to them.

### (A) The case where a "nucleic acid binding protein" is labeled with fluorescence

In this case, it is desirable that a length of a "nucleic acid" is made to be great so that a free nucleic acid binding protein is increased in molecular size thereof 5-fold or more at a time of formation of a complex bound to a nucleic acid. For example, when a transcription factor TFIID is used as a "nucleic acid binding protein", wherein a molecular weight thereof is 38 kDa, it is desirable that a "nucleic acid" to be bound is synthesized so as to have a molecular weight, preferably, of 152 kDa or more. Since in double-stranded DNA, one base pair has a molecular weight of 660 Da, DNA having a molecular weight of 152 kDa corresponds to 230 base pairs. On the other hand, in the case of a "nucleic acid" having a length of less than 230 base pairs, the binding of a protein and the nucleic acid can be analyzed by labeling each of the protein and the nucleic acid with two colors of fluorescence, and performing cross correlation analysis.

### (B) The case where "nucleic acid" is labeled

When a nucleic acid is labeled with fluorescence, the nucleic acid can be easily labeled by incorporating a fluorescent substance at an end upon synthesis of an oligonucleotide. Alternatively, a nucleic acid may be labeled utilizing a method (nick translation method) of treating an arbitrary nucleic acid with deoxyribonuclease I to make a nick, and adding four kinds of deoxyribonucleotides and DNA polymerase I to perform repair synthesis, wherein a fluorescent labeled nucleotide is incorporated into DNA upon the second reaction. Since the "nucleic acid binding protein" is considerably larger as compared with a synthetic oligonucleotide, a size of a nucleic acid is dramatically altered depending on complex formation, therefore, such complex formation can be identified by monomolecular fluorescent analysis.

As a marker for fluorescent labeling, any marker can be used as far as it is a substance emitting a signal which can be optically traced, that is, a substance which can be detected by light. For example, a fluorescent substance, a luminescent substance, an enzyme luminescent substance, and a radioactive substance can be used. Particularly, a fluorescent pigment is preferable because the pigment make it possible to individually measure the presence of micro substances. Preferably, an arbitrary fluorescent substance emitting detectable fluorescent light can be used. For example, various fluorescent pigments such as rhodamine, TAMRA, Cy3, Cy5 (Amersham), Alexa series (Molecular Probe), and fluorescent green protein GFP (Clontech) can be used. Fluorescent labeling can be performed by the known procedure.

A step of detecting binding of a "nucleic acid" and a "nucleic acid binding protein" is performed by placing the "nucleic acid" and the "nucleic acid binding protein" in a prescribed solution. The prescribed solution can be a solution in which a "nucleic acid" and a protein known to bind to the nucleic acid can be bound. For example, a physiological saline or a phosphate buffer can be employed.

By maintaining the "nucleic acid" and the "nucleic acid binding protein" under the prescribed condition in this prescribed solution, only a protein which can bind to the "nucleic acid" among "nucleic acid binding proteins" causes a binding reaction. Herein, the prescribed condition (temperature, pH, reaction time etc.) can be appropriately set depending on a kind of a "nucleic acid".

When the "nucleic acid binding protein" is labeled with fluorescence, an amount of the "nucleic acid binding protein" to be added to a reaction solution is such an amount that the final concentration becomes preferably about 0.01 to 100 nM, further preferably around 0.1 to 50 nM. An amount of the "nucleic acid" to be added is such an amount that the final concentration becomes preferably around 0.01 nM to 10 µM, further preferably around 0.1 to 50 nM.

As one example of a reaction, as mentioned in Examples described later, the reaction can be performed by mixing each 50 µL of a solution containing 10 nmol/L of the "nucleic acid" labeled with 5-TAMRA in a physiological buffered solution and a solution containing 1 to 100 nmol/L of the "nucleic acid binding protein" in a physiological buffered solution, and incubating them at room temperature for 15 to 30 minutes.

For incubation, a reaction solution containing a set of reaction components in the suspended state in the prescribed solution can be retained in an appropriate liquid retaining means such as a test tube, a well, a cuvette, a groove, a tube, a plate, and a porous medium. Herein, a shape, a material, and a size of the liquid retaining means are preferably selected so that a part or all of various detection steps such as dispensing, stirring, incubation, measurement, and conveyance are rapidly performed. For example, since measurement by monomolecular fluorescent analysis uses an extremely fine region at an optical focus level as a measurement place, the means can be a very small-type of liquid accommodating means. Particularly, in detection by optical measurement, the liquid retaining means preferably has an opening part for entrance and/or exit of a measuring beam so that the light beam for measurement is directly associated with reaction components as much as possible.

In a solution after the reaction, a presence ratio of the "nucleic acid binding protein" bound to the "nucleic acid" and the "nucleic acid binding protein" free in the reaction solution can be measured by monomolecular fluorescent analysis based on a difference in each molecular weight. Thereby, easiness (affinity) of binding of the "nucleic acid binding protein" to the "nucleic acid" can be obtained as a dissociation constant.

In addition, an antibody specific for a nucleic acid binding protein is added to the reaction solution after a binding reaction between the "nucleic acid" and the "nucleic acid binding protein", and thereby it can be reliably determined by further increase in a molecular weight that the nucleic acid binding protein is bound to nucleic acid (see Examples described later).

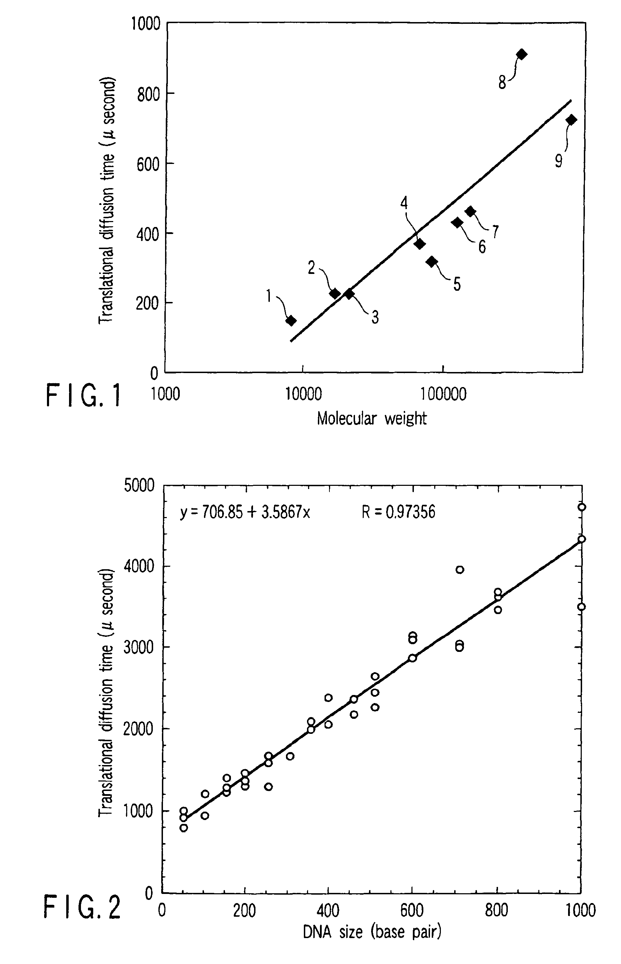

In addition, molecular weights of a nucleic acid and a nucleic acid binding protein can be easily calculated from a translational diffusion time obtained by analysis. FIG. 1 shows that a translational diffusion time is increased as a molecular weight of a protein is increased. In FIG. 1, an abscissa axis indicates a molecular weight of a protein, and an ordinate axis indicates a translational diffusion time (µ second). Reference numerals 1 to 9 shown in FIG. 1 denote following proteins.
1. α-Bungarotoxin, molecular weight 8000
2. Calmodulin, molecular weight 16700
3. Trypsin Inhibitor, molecular weight 21000
4. Bovine Serum Albumin, molecular weight 66000
5. Transferrin, molecular weight 80000
6. Lectin PHA-L from *Phaseolus vulgaris,* molecular weight 120000
7. IgG, molecular weight 150000
8. Fibrinogen, molecular weight 340000
9. α-Crystallin, molecular weight 800000

In addition, FIG. 2 shows that a translational diffusion time is increased as a DNA size (i.e. base pair) is increased. In FIG. 2, an abscissa axis indicates a length of a nucleotide sequence of DNA, and an ordinate axis indicates a translational diffusion time (µ second).

### <Apparatus for performing monomolecular fluorescent analysis>

An example of an apparatus for carrying out monomolecular fluorescent analysis (hereinafter, also referred to as fluorescent correlation analyzing apparatus) will be explained below by referring to FIG. 3.

As shown in FIG. 3, the fluorescent correlation analyzing apparatus is equipped with a laser light source 1; a light intensity regulating means (herein, ND filter) 2 for attenuating an intensity of a light beam 15 from the laser light source 1; a light attenuation selecting apparatus (herein, ND filter changer) 3 for setting an appropriate light intensity regulating means 2; a stage 6 on which a sample 14 containing a fluorescent molecule is placed; optical systems 4 and 5 for concentrating a light beam 15 from the laser light source 1 on the sample 14 to form a confocal region; optical systems 7 to 11 for concentrating the fluorescence emitted from the sample 14; a light detector 12 for detecting the concentrated fluorescence; and a fluorescent intensity recording means 13 for recording a change in a fluorescent intensity. As described, the fluorescent correlation analyzing apparatus utilizes a confocal laser microscope.

In FIG. 3, laser radiated from the laser light source 1 may be any of argon ion laser, helium-neon laser, krypton, and helium-cadmium. In FIG. 3, optical systems 4 and 5 for concentrating the light beam 15 from the laser light source on the sample to form a confocal region specifically mean a dichroic mirror 4, and an objective lens 5. The light beam 15 from the laser light source 1 proceeds through a route as shown by an arrow in FIG. 3. That is, an intensity of the light beam 15 from the laser light source 1 is first attenuated according to an attenuation degree of the fluorescent intensity regulating means (herein, ND filter) 2, then, the light beam 15 is refracted in a direction of a stage 90 degree relative to the incident light with the dichroic mirror 4, and irradiated on a sample on the stage 6 through the objective lens 5. In this way, the light beam is concentrated on the sample at fine one spot to form a confocal region.

In FIG. 3, optical systems 7 to 11 for concentrating the fluorescent light radiated from a fluorescent molecule in a confocal region specifically mean a filter 7, a tube lens 8, a reflection mirror 9, a pinhole 10, and a lens 11. The fluorescence radiated from a fluorescent molecule proceeds through a route shown with an arrow in FIG. 3. That is, the fluorescence radiated from a fluorescent molecule is first passed through the dichroic mirror 4 in a light progressing direction, refracted with the reflection mirror 9 via the filter 7 and the tube lens 8 to form an image on the pinhole 10, passed through the lens 11, and concentrated on a light detector 12.

The light detector (herein, avalanche photodiode) 12 for detecting the concentrated fluorescence converts the received light signal into an electric signal, and transmits it to a fluorescent intensity recording means (herein, computer) 13.

The fluorescent intensity recording means 13 for recording a change in a fluorescent intensity performs recording and analysis of transmitted fluorescent intensity data. Specifically, by analysis of the fluorescent intensity data, an autocorrelation function is set. Increase in a molecular weight due to binding of a fluorescent molecule to a receptor, and decrease in the number of free fluorescent molecules can be detected by a change in an autocorrelation function.

An apparatus for carrying out monomolecular fluorescent analysis (fluorescent correlation analysis apparatus) is not limited to the example shown in FIG. 3. For example, when a "nucleic acid" and a "nucleic acid binding protein" are discriminably labeled with two kinds of fluorescent substances having different excitation wavelengths, it is necessary that the fluorescent correlation analysis apparatus has two kinds of laser light sources for exciting respective fluorescent substances, and two kinds of light detectors for detecting the lights radiated from respective fluorescent substances. The light detector may be an apparatus comprising a photomultiplier tube in addition to APD (avalanche photodiode).

### <Regarding effect of the present invention>

Conventionally, measurement of transcription factor activity based on a force of binding of a transcription factor to a nucleic acid was performed by analysis using electrophoresis and an isotope (radioactive substance) such as a gel shift assay, an *in vitro* transcription assay and a footprinting method.

The gel shift assay utilizes reduction in mobility of electrophoresis of a DNA binding protein due to binding of a transcription factor and a particular DNA sequence, but since a large amount of a few mg of a protein is necessary, electrophoresis needs a time, and a molecular weight of a protein complex bound to DNA needs to be analyzed by autoradiography, much time and cost are required. In addition, even when there is a specific sample, it is difficult to recover the sample, and the assay is hardly extended to proteome analysis. In addition, much time, cost and labor are required in order to screen many proteins. Likewise, the *in vitro* transcription assay and the footprinting method need complicated operation of electrophoresis and autoradiography.

However, by the detection method using the monomolecular fluorescent analysis technique of the present invention, problems of the conventional methods have been solved, and the detection method of the present invention has advantages described below.
(1) Troublesomeness peculiar in the conventional methods is eliminated, and it is possible to rapidly and simply detect binding of a nucleic acid and a nucleic acid binding protein. Generally, it is enough that a measuring time of monomolecular fluorescent analysis is a few seconds to several tens of seconds, and measuring operation thereof is simple. In addition, a measuring cost can be suppressed low.
(2) The detection method of the present invention can detect not only the presence or the absence of binding of a nucleic acid binding protein to a nucleic acid, but also ability of a nucleic acid binding protein to bind to a nucleic acid (strength of binding force).
(3) Since the monomolecular fluorescent analysis technique is utilized, tens microliters of a sample solution is sufficient for the detection method of the present invention and, even when a concentration of a sample in a sample solution is low, detection with high sensitivity is possible. In addition, even when a sample solution is an unpurified crude solution, the solution can be used in the detection method of the present invention.
(4) Even when a crudely purified solution is used as a sample solution, and a molecular weight of a nucleic acid or a nucleic acid binding protein contained in the sample solution is unknown, a molecular weight can be calculated based on an increased value of a translational diffusion time which is measured as a result of a binding reaction.

To summarize the forgoing, according to the detection method of the present invention, all problems of the conventional procedure such as the gel shift assay, the *in vitro* transcription assay and the footprinting method can be solved. Inter alia, according to the method of the present invention, interaction between a nucleic acid and a protein exhibiting nucleic acid binding ability can be measured at a molecular level rapidly, with high sensitivity and a low cost. In addition, a molecular weight can be calculated from a translational diffusion time obtained by analysis.

### <Examples>

A synthetic oligonucleotide having a TATA box sequence was TAMRA-labeled, and behavior of formation of a complex of the oligonucleotide and transcription factors TFIID and TFIIB was analyzed in a solution. The results are shown in FIG. 4 and Table 1.

**Table 1**

| Sample | Molecular weight per molecule of complex (about kDa) | Actually measured value of translational diffusion time (µ second) | Predicted value of translational diffusion time (µ second) |
|---|---|---|---|
| 25-mer oligonucleotide of TFIID-binding site | 17 | 178 | 178 (Standard value) |
| 25-mer oligonucleotide of TFIID-binding site + TFIID protein | 55 | 237 | 264 |
| 25-mer oligonucleotide of TFIID-binding site + TFIID protein + TFIIB protein | 87 | 295 | 307 |
| 25-mer oligonucleotide of TFIID-binding site + TFIID protein + TFIIB protein + anti-TFIIB antibody | 227 | 435 | 423 |
| 21mer-oligonucleotide A | 14 | 208 | 167 |
| 21mer-oligonucleotide A + TFIID protein + TFIIB protein + anti-TFIIB antibody | 14 | 220 | 167 |

Fundamental nature of a transcription factor TFIID is according to the reference (Ehrenberg, M., Rigler, R.,; Chem. Phys., 4, 390-410, 1974). Fundamental nature of a transcription factor TFIIB is according to the reference (Patterson, M. G., et al.; Science, 248, 1625-1630, 1990).

A double-stranded DNA was obtained from a 25 base pairs oligonucleotide (Sigma Genosis) having a TFIID-binding site labeled with TAMRA, and human recombinant transcription factors TFIID and TFIIB (Promega) were added thereto sequentially. This labeled double-stranded DNA is referred to as "25-mer oligonucleotide of TFIID-binding site" in Table 1 and the following explanation. A concentration of the "25-mer oligonucleotide of TFIID-binding site" in a solution was set to be 5 nM, and concentrations of transcription factors TFIID and TFIIB in a solution were set to be 50 nM, respectively.

A sequence of the "25-mer oligonucleotide of TFIID-binding site" is shown as follows:
5'-GCA GAG CAT ATA AGG TGA GGT AGG A-3' (SEQ ID No.: 1)
3'-CGT CTC GTA TAT TCC ACT CCA TCC T-5' (SEQ ID No.: 2)

In FIG. 4, reference numeral (1) denotes a translational diffusion time of the oligonucleotide fluorescent molecule when a solution containing only the "25-mer oligonucleotide of TFIID-binding site" is used as a sample. Reference numeral (2) denotes a translational diffusion time of the fluorescent molecule in the solution when a transcription factor TFIID is added to the solution of (1). Reference numeral (3) denotes a translational diffusion time of the fluorescent molecule in the solution when a transcription factor TFIIB is added to the solution of (2). Reference numeral (4) denotes a translational diffusion time of the fluorescent molecule in the solution when an anti-TFIIB monoclonal antibody (Anti-TFIIB) is added to the solution of (3). Reference numeral (5) denotes a translational diffusion time of the oligonucleotide fluorescent molecule when a solution containing only a fluorescently labeled synthetic oligonucleotide (21 mer) having no TFIID-binding site is used as a sample. Reference numeral (6) denotes a translational diffusion time of the fluorescent molecule in the solution when a transcription factor TFIID, a transcription factor TFIIB, and an anti-TFIIB monoclonal antibody are added to the solution of (5). Additionally, in each case regarding the reference numerals (1) to (6), behavior of each molecule is schematically shown in FIG. 4.

The "25-mer oligonucleotide of TFIID-binding site" had a molecular weight of 17 kDa, and a translational diffusion time of 178 µ seconds (FIG. 4 (1)). It was seen that when TFIID is added to the oligonucleotide, a translational diffusion time becomes 237 µ seconds. Here, a translational diffusion time predicted from a molecular weight of the complex of 55 kDa is 264 µ seconds, and a difference between an actually measured value and a predicted value is within about 10% (FIG. 4 (2)). From this, it can be seen that a molecular weight can be estimated from a translational diffusion time.

Further, by addition of a transcription factor TFIIB, a complex becomes further great and its molecular weight becomes 87 kDa. It was seen that as a molecular weight per molecule is increased, a translational diffusion time (which is based on an original synthetic oligonucleotide as a standard) is increased and its value is approximate to a calculated value (FIG. 4 (3)). At this time, it was seen that a difference in a translational diffusion time between an actually measured value and a predicted value is within only about 4%.

Further, it was confirmed by addition of an anti-TFIIB or anti-TFIID monoclonal antibody that a complex is present in a solution (FIG. 4 (4)). The antibody has a molecular weight of 140 kDa, and a complex including the antibody has a molecular weight of 227 kDa. It was seen that a difference in a translational diffusion time between a predicted value and an actually measured value obtained likewise is within around 3%. Herein, as an antibody to be added to the solution, any of an anti-TFIIB antibody and an anti-TFIID antibody may be used, or both of them may be used.

As described above, reference numerals (1) to (4) in FIG. 4 show that, by successively binding proteins to the "25-mer oligonucleotide of TFIID-binding site", a size of the oligonucleotide fluorescent molecule is increased, and a translational diffusion time of the fluorescent molecule is increased.

On the other hand, in a synthetic oligonucleotide (21 mer) having no TATA box sequence, even when a transcription factor and an anti-transcription factor antibody is added, a complex having a fluorescent signal is not formed, and there was no remarkable increase in a translational diffusion time (Table 1, FIG. 4 (5) and (6)).

### Industrial Applicability

According to the present invention, a method of detecting binding of a nucleic acid and a nucleic acid binding protein by monomolecular fluorescent analysis is provided. Particularly, the present invention is useful in searching interaction between a gene and a transcription factor binding thereto, analyzing protein function, and detecting a drug utilizing it.

## Claims

1. A method for detecting binding of a nucleic acid and two kinds of nucleic acid binding proteins by monomolecular fluorescent analysis, **characterized by** comprising:
a step of detecting binding of a nucleic acid and a first nucleic acid binding protein; and
a step of, when binding of a nucleic acid and a first nucleic acid binding protein is detected in the above step, detecting binding of the resulting nucleic acid-protein complex and a second nucleic acid binding protein.

2. A method for detecting binding of a nucleic acid and n kinds (n indicates an integer of 2 or more) of nucleic acid binding proteins by monomolecular fluorescent analysis, **characterized by** comprising:
a step of detecting binding of a nucleic acid and a first nucleic acid binding protein; and
a step of, when binding of a nucleic acid and (X-1) kinds of first to (X-1)^{th} nucleic acid binding proteins is detected in the above step, repeating a step of detecting binding of the resulting nucleic acid-protein complex and a X^{th} nucleic acid binding protein (n-1) times sequentially from X = 2 to X = n.

3. The method according to claim 1 or 2, **characterized by** further comprising a step of confirming binding of a nucleic acid and one or more kinds of nucleic acid binding proteins using an antibody against any one of nucleic acid binding proteins.

4. The method according to any one of claims 1 to 3, **characterized in that t**he nucleic acid has a sequence of a protein-binding site.

5. The method according to any one of claims 1 to 4, **characterized in that** the nucleic acid binding protein is a TATA box binding protein.

6. The method according to any one of claims 1 to 5, **characterized in that** binding of the nucleic acid and the nucleic acid binding protein is detected with a light signal by binding the nucleic acid with a fluorescent substance, a luminescent substance, an enzyme luminescent substance, or a radioactive substance.

7. The method according to any one of claims 1 to 6, **characterized in that** the nucleic acid binding protein is a basal transcription factor, a transcription promoting factor, or a transcription inhibiting factor.

## Patentansprüche

1. Methode zum Nachweis der Bindung von Nukleinsäure und zwei Arten eines Nukleinsäure bindenden Proteins mittels monomolekularer Fluoreszenzanalyse, folgende Schritte umfassend:
Nachweis der Bindung von Nukleinsäure und einem ersten Nukleinsäure bindenden Protein; und
sofern im oberen Schritt der Nachweis von Nukleinsäure und einem ersten Nukleinsäure bindenden Protein erfolgt ist, Nachweis der Bindung des resultierenden Nukleinsäure-Protein-Komplexes und eines zweiten Nukleinsäure bindenden Proteins.

2. Methode zum Nachweis der Bindung von Nukleinsäure und n Arten (n bedeutet eine Ganze Zahl gleich oder größer 2) eines Nukleinsäure bindenden Proteins mittels monomolekularer Fluoreszenzanalyse, folgende Schritte umfassend:
Nachweis der Bindung von Nukleinsäure und eines ersten Nukleinsäure bindenden Proteins; und
sofern im oberen Schritt der Nachweis von Nukleinsäure und (X-1) Arten des ersten bis (X-1)ten Nukleinsäure bindenden Proteins erfolgt ist, die Wiederholung des Nachweises der Bindung des resultierenden Nukleinsäure-Protein-Komplexes und eines Xten Nukleinsäure bindenden Proteins (n-1) Mal in einer Sequenz von X = 2 bis X = n.

3. Methode gemäß Anspruch 1 oder 2, weiter umfassend die Bestätigung der Bindung von Nukleinsäure und eines oder mehrerer Arten von Nukleinsäure bindenden Proteinen mittels eines Antikörpers gegen jedes der Nukleinsäure bindenden Proteine.

4. Methode gemäß einem der Ansprüche 1 bis 3, **dadurch gekennzeichnet, dass** die Nukleinsäure eine Sequenz einer Protein-Bindungsstelle hat.

5. Methode gemäß einem der Ansprüche 1 bis 4, **dadurch gekennzeichnet, dass** das Nukleinsäure bindende Protein ein TATA-Box bindendes Protein ist.

6. Methode gemäß einem der Ansprüche 1 bis 5, **dadurch gekennzeichnet, dass** der Nachweis der Bindung der Nukleinsäure und des Nukleinsäure bindenden Proteins mittels Lichtsignal erfolgt, und zwar durch Bindung der Nukleinsäure an eine fluoreszierende Substanz, eine luminiszente Substanz , eine Enzym-luminiszente Substanz oder eine radioaktive Substanz.

7. Methode gemäß einem der Ansprüche 1 bis 6, **dadurch gekennzeichnet, dass** das Nukleinsäure bindende Protein ein basaler Transkriptionsfaktor, ein transkriptionsfördernder Faktor oder ein transkriptionshemmender Faktor ist.

## Revendications

1. Procédé de détection d'une liaison entre un acide nucléique et deux types de protéines de liaison aux acides nucléiques par analyse par fluorescence monomoléculaire, **caractérisé en ce qu'**il comprend :
une étape de détection d'une liaison entre un acide nucléique et une première protéine de liaison aux acides nucléiques ; et
lorsqu'une liaison entre un acide nucléique et une première protéine de liaison aux acides nucléiques est détectée à l'étape ci-dessus, une étape de détection d'une liaison entre le complexe acide nucléique-protéine résultant et une deuxième protéine de liaison aux acides nucléiques.

2. Procédé de détection d'une liaison entre un acide nucléique et n types (n représente un entier valant 2 ou plus) de protéines de liaison aux acides nucléiques par analyse par fluorescence monomoléculaire, **caractérisé en ce qu'**il comprend :
une étape de détection d'une liaison entre un acide nucléique et une première protéine de liaison aux acides nucléiques ; et
lorsqu'une liaison entre un acide nucléique et (X-1) types d'une première à une (X-1)^{ème} protéines de liaison aux acides nucléiques est détectée à l'étape ci-dessus, une étape de répétition d'une étape de détection d'une liaison entre le complexe acide nucléique-protéine résultant et une X^{ème} protéine de liaison aux acides nucléiques (n-1) fois séquentiellement de X = 2 à X = n.

3. Procédé selon la revendication 1 ou 2, **caractérisé en ce qu'**il comprend en outre une étape de confirmation d'une liaison entre un acide nucléique et un ou plusieurs types de protéines de liaison aux acides nucléiques, utilisant un anticorps contre l'une quelconque des protéines de liaison aux acides nucléiques.

4. Procédé selon l'une quelconque des revendications 1 à 3, **caractérisé en ce que** l'acide nucléique a une séquence d'un site de liaison aux protéines.

5. Procédé selon l'une quelconque des revendications 1 à 4, **caractérisé en ce que** la protéine de liaison aux acides nucléiques est une protéine de liaison à la boîte TATA.

6. Procédé selon l'une quelconque des revendications 1 à 5, **caractérisé en ce qu'**une liaison entre l'acide nucléique et la protéine de liaison aux acides nucléiques est détectée avec un signal lumineux par la liaison de l'acide nucléique avec une substance fluorescente, une substance luminescente, une substance luminescente enzymatique, ou une substance radioactive.

7. Procédé selon l'une quelconque des revendications 1 à 6, **caractérisé en ce que** la protéine de liaison aux acides nucléiques est un facteur de transcription de base, un facteur stimulant la transcription, ou un facteur inhibant la transcription.
